(19) Europäisches Patentamt
European Patent Office
Office européen des brevets

(11) EP 0 473 308 B1

(12) **EUROPEAN PATENT SPECIFICATION**

(45) Date of publication and mention of the grant of the patent:
**22.05.1996 Bulletin 1996/21**

(51) Int. Cl.[6]: **C07C 65/24**, C07C 65/26, A61K 31/19

(21) Application number: **91307304.5**

(22) Date of filing: **08.08.1991**

(54) **Aldose reductase inhibitor**

Hemmungsstoff für Aldosereduktase

Inhibiteur d'aldose réductase

(84) Designated Contracting States:
**AT BE CH DE DK ES FR GB GR IT LI LU NL SE**

(30) Priority: **08.08.1990 JP 208128/90**

(43) Date of publication of application:
**04.03.1992 Bulletin 1992/10**

(73) Proprietor: **ASAHI KASEI KOGYO KABUSHIKI KAISHA**
**Osaka (JP)**

(72) Inventors:
- **Suzuki, Yukio**
  **Tagata-gun, Shizuoka (JP)**
- **Kuno, Kouichi**
  **Tagata-gun, Shizuoka (JP)**
- **Shoda, Motoshi**
  **Tagata-gun, Shizuoka (JP)**
- **Yaso, Masao**
  **Tagata-gun, Shizuoka (JP)**
- **Yaginuma, Satoshi**
  **Tagata-gun, Shizuoka (JP)**
- **Asahi, Akira**
  **Mishima-shi, Shizuoka (JP)**

(74) Representative: **Woods, Geoffrey Corlett et al**
**J.A. KEMP & CO.**
**14 South Square**
**Gray's Inn**
**London WC1R 5LX (GB)**

(56) References cited:
**US-A- 4 749 571**

- **Journal of the Indian Chemical Society, vol. 42, no. 2, February 1965, Indian Chemical Society, Calcutta, pages 86 - 90; K.P. Mathai et al.: "Synthesis of bitetralonyl and bianthronyl derivatives from biphenyl derivatives"**
- **PATENT ABSTRACTS OF JAPAN, vol. 5, no. 204 (C-85)(876), 24 December 1981, & JP-A-56 123 935**
- **PATENT ABSTRACTS OF JAPAN, vol. 11, no. 132 (C-417)(2579), 24 April 1987, & JP-A-61 268 182**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 473 308 B1

## Description

This invention relates to an aldose reductase inhibitor.

Among biphenyl derivatives having an aldose reductase inhibiting activity there are known aldostatin (as disclosed in JP-A-205095/1987) and FR 900280 (as disclosed in JP-A-72144/1990). These compounds are now under examination for their application to prevent or treat diabetic complications, such as cataracts, retinal disease, neuropathy and nephropathy.

Nevertheless, there is still a demand for a substance having a higher aldose reductase inhibiting activity than these.

We have found an aldose reductase inhibiter having superior inhibiting activity.

The present invention provides an aldose reductase inhibitor which comprises a compound of formula (I):

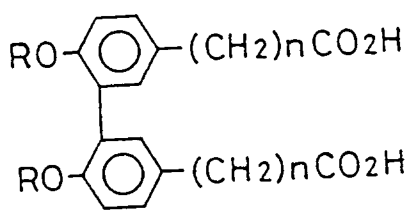

in which each R, which may be the same or different, is a $C_1$-$C_6$, preferably $C_1$-$C_4$, alkyl or cyclohexylmethyl group and each n, which may be the same or different, is 2 or 3, or a physiologically acceptable salt thereof, and a carrier.

The present invention also provides a compound of formula (I) or a physiologically acceptable salt thereof, with the proviso that when the compound is in the form of the free acid, and both R groups are methyl groups, both ns are not 3.

The present invention further provides a process for the preparation of a compound of formula (I), which comprises hydrolysing a compound of formula (II):

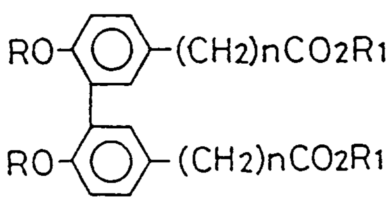

wherein R and n are as defined above and each $R_1$, which may be identical or different, is a radical removable by hydrolysis and, if desired, converting the compound of formula (I) thus produced to a physiologically acceptable salt thereof.

The present invention yet further provides a compound of formula (I) or a physiologically acceptable salt thereof for use in a method of treatment of the human or animal body by therapy, especially for use in a method of treatment of a condition requiring an aldose reductase inhibitor.

The present invention still further provides the use of a compound of formula (I) or a physiologically acceptable salt thereof in the manufacture of a medicament for the treatment of a condition requiring an aldose reductase inhibitor.

The present invention additionally provides a process for producing an aldose reductase inhibitor which comprises mixing together a compound of formula (I) or a physiologically acceptable salt thereof and a carrier.

R is preferably an alkyl group having from 1 to 5 carbon atoms or a cyclohexylmethyl group.

Of the compounds of formula (I), the compound in which both Rs denote methyl and both ns are 3 is known (Indian Chem. Soc. 42, 86 (1965)), but no pharmacological use has been reported. While the compounds of formula (I) can be produced in a similar way as described in the above reference, it is also possible to prepare them by the reaction scheme given below:

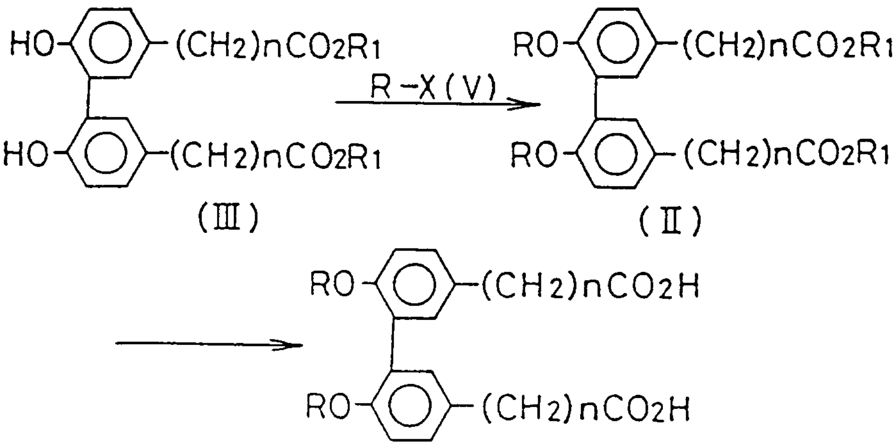


in which R and n are as defined above, each $R_1$ is a radical removable by hydrolysis and X is a halogen atom, by alkylating the phenolic hydroxyl groups of an esterified biphenyl dicarboxylic compound of formula (III) using an alkyl halide of formula (V) to produce the compound of formula (II) and then converting the ester group of the compound of formula (II) into the free acid group to prepare the compound of formula (I).

Examples of the alkyl halide of formula (V) to be used for alkylating the esterified compound of formula (III) are alkyl iodides and alkyl bromides having 1 - 5 carbon atoms and cyclohexylmethyl bromide.

The alkylation can be realized in an organic solvent which will not participitate in the reaction, such as DMF, acetone, dioxane, tetrahydrofuran, benzene, toluene, xylene, ethyl acetate, methanol or ethanol, by introducing therein the esterified compound of formula (III) and the alkyl halide of formula ( V) and conducting the reaction in the presence of an inorganic or organic base, for example, an alkali metal carbonate, such as, anhydrous potassium carbonate or anhydrous sodium carbonate; an alkali metal hydroxide, such as, sodium or potassium hydroxide; a tertiary alkylamine, such as, trimethylamine or triethylamine; pyridine or a pyridine derivative, such as, picoline, lutidine or 4-dimethylaminopyridine; a diazabicyclo compounds, such as, 1,5-diazabicyclo [4.3.0]-nonene-5, 1,4-diazabicyclo [2.2.2]-octane, 1,8-diazabicyclo [5.4.0] - undecene-7; and a metal alkoxide, such as, sodium methoxide or sodium ethoxide, together with, if necessary, a small amount of copper powder as catalyst.

The reaction is carried out in general at room temperature, or under heating to 60 - 100°C for poorly reactive compounds, for a duration of 2 - 6 days under agitation. The amount of the reagents and catalyst may preferably be in the range from 5 to 40 moles of the halide, from 2 to 7 of the base and from 0.5 to 1.5 times by weight of the catalyst per mole of the starting compound.

Among the starting compounds, namely, the esterified biphenyl compounds of formula (III), the compound in which n is 2 is known [(J. Org. Chem. 46, 1991 (1981)] and the compound in which n is 3 (IIIb) can be prepared easily as shown in the following reaction scheme

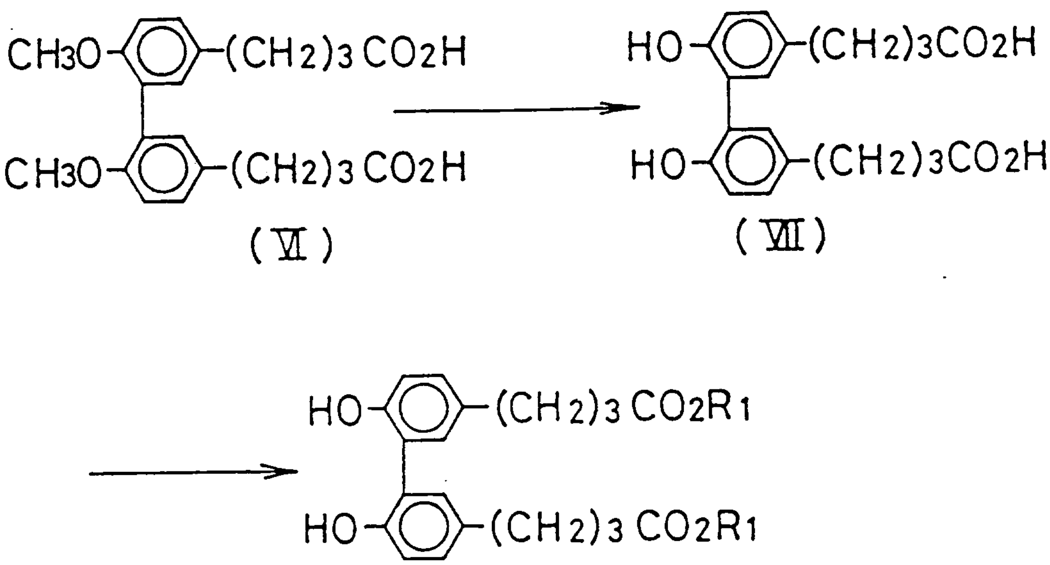

(VI) (VII) (IIIb)

by reacting pyridine-hydrochloric acid with a known compound of formula (VI) at about 180°C to convert the methoxy group into the hydroxide group and, then, esterifying the carboxyl groups of the resulting product of formula (VII) with, for example, a $C_{1-6}$, preferably a $C_{1-4}$, alcohol.

The ester splitting, namely, hydrolysis of the ester groups of the resulting compound of formula (II) can be effected by reacting the ester in an organic solvent, such as, ethanol, methanol, dioxane, tetrahydrofuran or acetonitrile, with a base, such as, sodium hydroxide or potassium hydroxide for 1 - 4 days at room temperature under agitation. The concentration of the base to be employed may in general be in the range from 1 to 3 N and it is preferable to use it in an amount of about 8 moles per mole of the compound of formula (II).

The compound of formula (I) according to the present invention obtained as above can further be purified on requirement using any known purifying means, such as, column chromatography on silica gel and recrystallization.

The compound of formula (I) according to the present invention can be converted into a corresponding physiologically acceptable salt of an inorganic cation of, for example, an alkali metal, such as, sodium or potassium; an alkaline earth, such as, calcium or magnesium; and ammonium and of an organic cation of, for example, a known organic amine. For preparing an inorganic salt, the compound of formula (I) according to the present invention is preferably dissolved first in an aqueous solution containing at least an equivalent amount of hydroxide, carbonate or of a metal corresponding to the contemplated inorganic salt. For such a salt formation reaction with a metal compound, a water-miscible organic solvent inert to the reaction, such as, methanol, ethanol, acetone and dioxane may be admixed to the reaction mixture. If sodium hydroxide, sodium carbonate or sodium bicarbonate is employed, a solution of the corresponding sodium salt will be obtained.

A solid salt can be obtained by evaporating off the solvent of such a solution or by adding to the solution a water-soluble organic solvent having some polarity, such as, butanol, or ethyl methyl ketone, to cause deposition of the solid salt.

The compound of formula (I) according to the present invention or the salt thereof can be used for an aldose reductase inhibitor as such or in a formulation with a known carrier substance.

The aldose reductase inhibitor according to the present invention can be administered to a patient or to an experimental animal, for example, orally, rectally or parenterally, such as, by intraveous, intramuscular, subcutaneous and intraperitoneal administration and by eye wash. The preparation of the compound according to the present invention may be formulated so as to adapt to each of the above administration techniques.

The preparation may be in a form of, for example, pellet, pill, powder, granule, capsule, suppository, injection, preparation and eye wash. For formulating such preparations for oral administration, such as, tablet, granule and encapsulated drug, the compound according to the present invention may be mixed with, for example, an excipient, such as a pertinent carrier substance, such as, strach, lactose, sucrose, mannite, carboxymethyl cellulose, corn starch and various inorganic salts; a binder, such as, starch, dextrin, gum arabic, gelatine, hydroxypropyl starch, methyl cellulose, sodium carboxymethyl cellulose, hydroxypropyl, cellulose, crystallized cellulose, ethyl cellulose, polyvinyl pyrrolidone and macrogol; a crumbilizer, such as, starch, hydroxypropyl starch, carboxymethyl cellulose, sodium cerboxymethyl cellulose and

hydroxypropyl cellulose; a surface active agents, such as, sodium lauryl sulfate, soy been lecithin, a fatty acid ester of sucrose and polysorbate 80; a lubricating agent, such as, talc, wax, a hydrogenated vegetable oil, a fatty acid ester of sucrose, magnesium stearate and calcium stearate; a fluidizability promotor; a taste modifier; a colouring agent and flavors.

The compound of formula (I) according to the present invention or a salt thereof can be administered in a form of suspension, emulsion, syrup or elixir.

For parenteral preparations, there may be incorporated, in general, distilled water for use for injection, physiological saline solution, aqueous solution of glucose, vegetable oils for use for injection, propylene glycol, polyethylene glycol as the diluent. It is permissible to further incorporate on requirement, for example, a sterilizer, an antiseptic or a stabilizer. It is possible to process such parenteral preparations in consideration of their poor stability into freeze-dried products by subjecting the preparation placed in a suitable vessel, such as a glass vial, to freeze-drying, so as to permit the reproduction of the preparation directly before its use by diluting it with a pertinent diluent. It is also, for example, possible to incorporate into the preparation on requirement an isotonating agent, a stabilizer, an aspectic or an analgesic. The administration dose of the compound of formula (I) or the salt thereof according to the present invention may vary in accordance with each selected administration course and with the condition of the subject to be administered, such as, age, body weight, disease condition and so on. It is in general in the range of from 5 milligrams to 1 gram, preferably in the range of from about 25 milligrams to 1 gram, more preferably in the range of from about 25 milligrams to about 300 milligrams, calculated as the compound of formula [I], per day per adult, by an administration of once a day or in two or three daily aliquotes.

Various compounds of formula (I) according to the present invention have been examined for their pharmacodynamic behavior and for their acute toxicity, of which results are given below.

1) METHOD FOR DETERMINING THE ENZYME REACTION INHIBITING ACTIVITY

The preparation of the aldose reductase and the determination of the enzyme reaction inhibiting activity were carried out pursuant to the procedures disclosed by Haymann et al in the literature Journal of Biological Chemistry 240, 877 (1965).

The sample solution to be examined for the inhibiting activity was prepared by dissolving an amount of the compound of formula (I) according to the present invention in a small amount of methanol and supplementing the resulting methanol solution by diluting with distilled water up to tenfold volume. The enzyme, i.e. aldose reductase, which originated from the lens of cattle was employed after being subjected to a partial purification.

To 2.4 ml of $5 \times 10^{-5}$ M NADPH solution dissolved in a 2/15 M phosphate buffer solution (pH = 6.0), 0.4 ml of the enzyme solution and 0.1 ml of the sample solution to be examined were added and the mixture was charged in a quartz cell of a photoelectric photometer and was subjected to a pre-incubation at 37°C for 4 minutes. Then, 0.1 ml of a 0.015 M D,L-glyceraldehyde solution was added thereto for initiating the enzyme reaction and the decrease in the photoabsorbance at 340 nm was pursued at 37°C for 4 minutes. The aldose reductase inhibiting activity was calculated by the formula :

$$\frac{B-A}{B} \times 100\ (\%)$$

in which A represnts the enzyme reaction rate with addition of the inhibitor and B denotes the enzyme reaction rate without addition of the inhibitor.

2) ACTIVITY FOR SUPPRESSING ACCUMULATION OF SORBITOL

(A) Tissue Culture

Lenses and sciatic nerves excised from 7 week-old male Wistar rats were washed twice with an aseptic buffer solution consisiting of 0.3 % trisaminomethane, 0.8 % NaCl, 0.038 % KCl, 0.025 % $Na_2HPO_4 \cdot 12H_2O$, 0.1 % glucose and 1.85 ml/l of concentrated HCl.

The lenses and the sciatic nerves were then treated by a 30 minutes preculture in a culture medium prepared by adding 2 ml of 7.5 % conc. aseptic $NaHCO_3$ solution and 5.5 ml of aseptic calf fetus serum to 100 ml of an aseptic culture medium consisting of 0.98 % TC Medium 199 (supplied from the firm NISSUI) containing 0.01 % of dihydrostreptomycin and 0.01 % of benzylpenicilin. Thereafter, the intrinsic culture was effected in a culture medium prepared by adding each sample solution to be examined and 0.09 % of glucose to the abovementioned culture medium for 20 hours. The culture was effected at 37°C in an atmosphere composed of 95 % of air and 5 % of $CO_2$.

After the culture, the cultured samples of lenses and sciatic nerves were taken out of the culture medium onto a filter paper and the weight of each of them was measured after the adherent culture solution had been swept off. Then, each of the cultured samples was homogenized with the addition of 1 ml of a cold 8 % $HClO_4$ solution and the homog-

enized mixture was subjected to a centrifugation at 10,000 G for 10 minutes, whereby a supernatant was obtained. The resulting precipitate was then homogenized again with the addition thereto of 0.5 ml of a cold 8 % $HClO_4$ solution and the homogenized mixture was again subjected to a centrifugation in a similar way as above to obtain a further supernatant. This was put together with the previously obtained supernatant and the resulting solution was neutralized with 2 N KOHaq. solution and was supplemented with distilled water to a volume of 5 ml. The precipitate formed by the neutralization was separated by a centrifugation at 3,000 rpm for 10 minutes and the resulting supernatant was employed as the sample for determining the sorbitol content.

(B) Determination of Sorbitol Content

The determination of the sorbitol content in the lens and in the sciatic nerve was carried out in accordance with the technique disclosed by Bergmeyer in "In Methods of Enzyme Analysis" edited by H.U. Bergmeyer, pp 1323 - 1326, Academic Press, New York, 1974 and in accordance with the technique proposed by Malaon et al in "Diabetes" 29, 861 - 864 (1980).

To 0.5 ml of the sample solution for determining the sorbitol content obtained as above, 0.9 ml of 0.1 M glycine - KOH buffer solution (pH 9.4) containing 1 mM of $NAD^+$ and 0.1 ml of a solution containing 20 Units/ml sorbitol dehydrogenase were added and the resulting mixture was subjected to incubation at 37°C for 1 hour.

The content of sorbitol in each tissue was estimated by assuming the increment of the photoabsorbance at 340 nm to be proportional to the amount of NADH produced by the reaction for the lens and by detecting the fluorescence with an exitation wavelength of 366 nm and a fluorescence wavelength at 425 nm for the sciatic nerve.

The activity for suppressing accumulation of sorbitol, or the aldose reductase inhibiting activity, was calculated from the determined sorbitol content by the formula:

$$\frac{(B-C)-(A-C)}{(B-C)} \times 100\ (\%)$$

in which A denotes the content of sorbitol per unit weight of the tissue after cultivation with addition of the inhibitor, B denotes the sorbitol content per unit weight of the tissue after cultivation without addition of the inhibitor and C represents the sorbitol content per unit weight of the tissue after cultivation in the medium without addition of glucose and inhibitor.

The results of the tests according to 1) and 2) above are summarized in Table 1 below. Here, it is to be pointed out that the diester intermediate of formula (II) used in the production of the compound of formula (I) according to the present invention and the objective products obtained in Reference Examples 1 - 8 given afterwards as well as the compound 2,2'-dimethoxy-5,5'- bis(3-carboxypropyl)biphenyl diester did not in themselves exhibit any aldose reductase inhibiting activity and any activity for suppressing accumulation of sorbitol.

Table 1

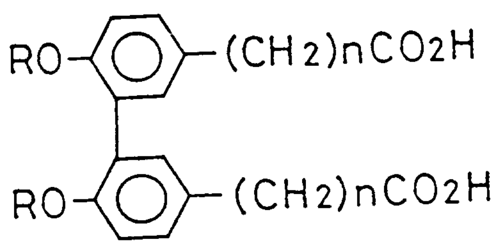

(I')

| Compound of formula(I') | | Enzyme Reaction Inhibiting Activity (%) [1] | Inhibition of Sorbite Accumulat. in Lens (%) [2] | Inhibition of Sorbite Accum. in Sciatic N. (%) [3] |
|---|---|---|---|---|
| R | n | | | |
| $CH_3$ – | 2 | 44 | 76 | 85 |
| $C_2H_5$ – | 2 | 43 | 56 | 69 |
| $C_3H_7$ – | 2 | 35 | 66 | 67 |
| 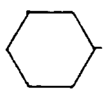 -$CH_2$- | 2 | 44 | 51 | – |
| $CH_3$ – | 3 | 63 | 85 | 92 |
| $C_2H_5$ – | 3 | 77 | 76 | 80 |
| $C_3H_7$ – | 3 | 57 | 68 | 67 |
| 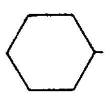 -$CH_2$- | 3 | 45 | 57 | – |
| $C_5H_{11}$ – | 3 | 48 | – | – |

Notes:

1) Tests were carried out at a concentration of 0.1 γ.

2) Tests were carried out at a concentration of 2.5 γ.

3) Tests were carried out at a concentration of 1.0 γ.

3) ACUTE TOXICITY VALUE

An amount of the compound of formula (I) according to the present invention, for example those given in Example 1 - 8 below or 2,2'-dimethoxy-5,5'-bis(3-carboxypropyl)biphenyl, was suspended in distilled water and was dissolved completely by adding at least an equimolar amount of aqueous NaOH solution. The pH of the resulting solution was adjusted to 6.5 - 7.0 and the solution was administered to groups of 5 weeks old STD-DDY mice (each group consisted of 3 mice) orally at a rate of 400 mg/20 ml/kg and each group was observed for 10 days. No fatality occurred in any of the administered groups.

As is clear from the experimental results, the compounds according to the present invention exhibit a superior aldose redusctase inhibition activity and excellent stability, so that they can be employed advantegeously as aldose reductase inhibitors for, for example, the prevention or therapy of cataracts, retinal diseases, neuropathy, nephropathy, corneal disorders and diabetic uveitis.

The present invention will now be further described in the following Examples and Reference Examples.

Reference Example 1

Synthesis of 2,2'-Dimethoxy-5,5'-bis(2-ethoxycarbonylethyl)biphenyl

To 5 ml of a DMF solution containing 200 mg(0.05181 mmol) of 2,2'-dihydroxy-5,5'-bis(2-ethoxycarbonylethyl)biphenyl and 2.316 ml (20.72 mmol) of methyl iodide, there were added 572 mg (2.072 mmol) of anhydrous potassium carbonate and a small amount of copper powder and the resulting mixture was agitated for 4 days at room temperature and for a further 2 days at 60°C. The reaction mixture was filtered with suction through Celite to remove the solid matter, which was washed with ethyl acetate. After the solvent in the filtrate was evaporated off under reduced pressure, the evaporated residue was dissolved in an amount of chloroform. This solution was washed with dilute hydrochloric acid, dilute aqueous sodium bicarbonate, water and saline water in this sequence and was dried over anhydrous sodium sulfate. The residue obtained after the solvent had been evaporated off was purified by silica gel column chromatography (with 20 g of WAKO-gel C-200, eluent: toluene and toluene/ethyl acetate of 50/l) to obtain 182 mg (0.4936 mmol, 85 %) of 2,2'-dimethoxy-5,5'-bis(2-ethoxycarbonylethyl)biphenyl as an oily product.

$^1$H-NMR ($CDCl_3$)

δ (ppm) :

1.24 (6H、t、2$CH_3$)、2.61 (4H、t、2$CH_2$)、2.92 (4H、t、2$CH_2$)、3.74 (6H、s、2$CH_3$)、4.13 (4H、q、2$CH_2$)、6.8-7.2 (6H、m、arom-H)

MS (FAB) : 414 ($M^+$)

Reference Example 2

Synthesis of 2,2'-Diethoxy-5,5'-bis(2-ethoxycarbonylethyl)biphenyl

To an ethanol solution containing 200 mg (0.518 mmol) of 2,2'-dihydroxy-5,5'-bis(2-ethoxycarbonylethyl), 2.18 ml (1.1398 mmol) of a 0.5217 N ethanol solution of sodium ethoxide were added and the solution was evaporated off. The evaporated residue was dried sufficiently and was dissolved in 5 ml of DMF, to which was added 0.211 ml (2.5905 mmol) of ethyl iodide and the mixture was agitated at room temperature for 4 hours. There was further added thereto a DMF suspension containing 28.2 mg (0.4144 mmol) of sodium ethoxide and the resulting mixture was agitated for 1 hour. The solvent of the reaction mixture was evaporated off under reduced pressure and, to the resulting evaporation residue, an amount of chloroform was added. The solution obtained was washed three times with table salt water and was dried

over anhydrous sodium sulfate, before the solvent was evaporated off. The resulting evaporated residue was subjected to a purification with silica gel column chromatography (20 g of WAKO-gel C-200, eluent: toluene and toluene/ethyl acetate of 100/l) to obtain 188.2 mg (0.4258 mmol), 82 %) of 2,2'-diethoxy-5,5'-bis(2-ethoxycarbonylethyl)biphenyl as an oily product.

$^1$H-NMR ($CDCl_3$)

δ (ppm) :

1.23 (6H、t、$2CH_3$)、1.25 (6H、t、$2CH_3$)、2.61 (4H、t、$2CH_2$)、2.91 (4H、t、$2CH_2$)、3.97 (4H、q、$2CH_2$)、4.13 (4H、q、$2CH_2$)、6.8-7.2 (6H、m、arom-H)

MS (FAB) : 442 ($M^+$)

Reference Example 3

Synthesis of 2,2'-Dipropoxy-5,5'-bis(2-ethoxycarbonylethyl)biphenyl

To an ethanol solution containing 200 mg (0.518 mmol) of 2,2'-dihydroxy-5,5'-bis(2-ethoxycarbonylethyl), 3.78 ml (2.0724 mmol) of a 0.5478 N ethanol solution of sodium ethoxide were added and the solvent was evaporated off. The evaporation residue was dried sufficiently and was dissolved in 5 ml of DMF. Thereto was added 0.238 ml (2.591 mmol) of propyl bromide and the mixture was agitated at room temperature for 15 hours, whereupon 0.255 ml (2.591 mmol) of propyl iodide was added thereto. After three hours, a DMF suspension contaning 70.5 mg (1.0362 mmol) of sodium ethoxide was added thereto and the resulting mixture was agitated for 4 hours. The solvent of the reaction mixture was evaporated off under reduced pressure and the resulting evaporated solution was washed three times with saline water and was dried over anhydrous sodium sulfate, before the solvent was evaporated off. The resulting evaporated residue was subjected to a purification with silica gel column chromatography (50 g of WAKO-gel C-200, eluent: hexane and hexane/ethyl acetate of 60/l, 40/l, 35/l. 35/l, 30/l and 25/l) to obtain 129 mg (0.2742 mmol, 53 %) of 2,2'-dipropoxy-5,5'-bis(2-ethoxycarbonylethyl)biphenyl as an oily product.

$^1$H-NMR ($CDCl_3$)

δ (ppm) :

0.85 (6H、t、$2CH_3$)、1.23 (6H、t、$2CH_3$)、1.63 (4H、m、$2CH_2$)、2.4-2.7 (4H、m、$2CH_2$)、2.7-3.0 (4H、m、$2CH_3$)、3.83 (4H、t、$2CH_2$)、4.12 (4H、q、$2CH_2$)、6.7-7.2 (6H、m、arom-H)

MS (FAB) : 470 ($M^+$)

Reference Example 4

Synthesis of 2,2'-Dicyclohexylmethoxy-5,5'-bis(2-ethoxycarbonylethyl)biphenyl

To 5 ml of a DMF solution containing 200 mg (0.518 mmol) of 2,2'-dihydroxy-5,5'-bis(2-ethoxycarbonylethyl) biphenyl and 0.730 ml (5.181 mmol) of cyclohexylmethyl bromide, there were added 286 mg (2.0724 mmol) of anhydrous potassium carbonate and a small amount of copper powder and the resulting mixture was agitated for 24 hours at room temperature. The reaction mixture was filtered with suction through Celite to remove the solid matter, which was washed with ethyl acetate. After the solvent in the filtrate was evaporated off under reduced pressure, the residue was dissolved in an amount of chloroform. This solution was washed with dilute hydrochloric acid, dilute aqueous sodium bicarbonate, water and saline water in this sequence and was dried over anhydrous sodium sulfate. The residue obtained after the solvent had been evaporated off was purified by silica gel column chromatography (with 20 g of WAKO-gel C-200, eluent: hexane and hexane/ethyl acetateof 25/l) to obtain 91 mg (0.1574 mmol, 30 %) of 2,2'-dicyclohexylmethoxy-5,5'-bis(2-ethoxycarbonylethyl)-biphenyl as an oily product.

$^1$H-NMR ($CDCl_3$)

δ (ppm) :

0.8-1.8 (22H、m、cyclohexyl-H)、1.24 (6H、t、$2CH_3$)、2.58 (4H、t、$2CH_2$)、2.89 (4H、t、$2CH_2$)、3.65 (4H、d、$2CH_2$)、4.13 (4H、q、$2CH_2$)、6.8-7.2 (6H、m、arom-H)

MS (FAB) : 578 ($M^+$)

Example 1

Synthesis of 2,2'-Dimethoxy-5,5'-bis(2-carboxyethyl)biphenyl

To 10 ml of an ethanol solution containing 170 mg (0.4111 mmol) of 2,2'-dimethoxy-5,5'-bis(2-ethoxycarbonylethyl)biphenyl obtained in Reference Example 1, 3.3 ml (3.3 mmol) of a 1 N solution of sodium hydroxide were added and the mixture was agitated at room temperature for 24 hours. The solvent of the reaction mixture was evaporated off under reduced pressure and, to the resulting evaporation residue, an amount of water was added. The solution obtained

was acidified by adding 1 N hydrochloric acid to a pH of 1 - 2. The thus formed precipitate was filtered, washed with water and dried, whereby 145 mg (0.4056 mmol, 99 %) of the title compound were obtained as a white solid.

$^1$H-NMR ($CDCl_3$)

δ (ppm) :

2.58 (4H、t、$2CH_2$)、2.87 (4H、t、$2CH_2$)、3.70 (6H、s、$2CH_3$)、6.9-7.2 (6H、m、arom-H)

MS (FAB) : 357 $(M-H)^-$

Example 2

Synthesis of 2,2'-Diethoxy-5,5'-bis(2-carboxyethyl)biphenyl

To 10 ml of an ethanol solution containing 172 mg (0.3891 mmol) of 2,2'-diethoxy-5,5'-bis(2-ethoxycarbonylethyl)biphenyl obtained in Reference Example 2, 3.1 ml (3.1 mmol) of 1 N sodium hydroxide solution were added and the mixture was agitated at room temperature overnight. The solvent of the reaction mixture was evaporated off under reduced pressure and, to the resulting evaporation residue, an amount of water was added. The solution obtained was acidified by adding 1 N hydrochloric acid to a pH of 1 - 2. The thus formed precipitate was filtered, washed with water and dried, whereby 129.6 mg (0.3358 mmol, 86 %) of the title compound were obtained as a white solid.

$^1$H-NMR ($CDCl_3$)

δ (ppm) :

1.28 (6H、t、$2CH_3$)、2.59 (4H、t、$2CH_2$)、2.95 (4H、t、$2CH_2$)、4.01 (4H、q、$2CH_2$)、6.8-7.2 (6H、m、arom-H)

MS (FAB) : 387 $(M+H)^+$

Example 3

Synthesis of 2,2'-Dipropoxy-5,5'-bis(2-carboxyethyl)biphenyl

To 7 ml of an ethanol solution containing 182.9 mg (0.2742 mmol) of 2,2'-dipropoxy-5,5'-bis(2-ethoxycarbonylethyl)biphenyl obtained in Reference Example 3, 2.2 ml (2.2 mmol) of 1 N sodium hydroxide solution were added and the mixture was agitated at room temperature for 2 days. The solvent of the reaction mixture was evaporated off under reduced pressure and, to the resulting evaporation residue, an amount of water was added. The solution obtained was acidified by adding 1 N hydrochloric acid to a pH of 1 - 2. The thus formed precipitate was filtered, washed with water and dried, whereby 108.4 mg (0.2618 mmol, 95 %) of the title compound were obtained as a white solid.

$^1$H-NMR (MeOH-$d_4$)

δ (ppm) :

0.89 (6H、t、$2CH_3$)、1.64 (4H、m、$2CH_2$)、2.4-2.7 (4H、m、$2CH_2$)、2.7-3.0 (4H、m、$2CH_2$)、3.86 (4H、t、$2CH_2$)、6.8-7.2 (6H、m、arom-H)

MS (FAB) : 413 $(M-H)^-$

Example 4

Synthesis of 2,2'-Dicyclohexylmethoxy-5,5'-bis(2-carboxyethyl)biphenyl

To 3.6 ml of an ethanol solution containing 86.7 mg (0.1500 mmol) of 2,2'-dicyclohexylmethoxy-5,5'-bis(2-ethoxycarbonylethyl)biphenyl obtained in Reference Example 4, there were added 1.2 ml (1.2 mmol) of 1 N sodium hydroxide solution and the mixture was agitated at room temperature. The solvent of the reaction mixture was evaporated off under reduced pressure and, to the resulting evaporated residue, an amount of water was added. The solution obtained was acidified by adding 1 N hydrochloric acid to a pH of 1 - 2. The thus formed precipitate was filtered, washed with water and dried, whereby 75.9 mg (0.1454 mmol, 97 %) of the title compound were obtained as a white solid.

$^1$H-NMR ($CDCl_3$)

δ (ppm) :

0.8-1.8 (22H、m、cyclohexyl-H)、2.59 (4H、t、$2CH_2$)、2.93 (4H、t、$2CH_2$)、3.66 (4H、d、$2CH_2$)、6.7-7.2 (6H、m、arom-H)

MS (FAB) : 523 $(M+H)^+$

Reference Example 5

Synthesis of 2,2'-Diethoxy-5,5'-bis-(3-methoxycarbonylpropyl)biphenyl

To a methanol solution containing 255 mg(0.6617 mmol) of 2,2'-dihydroxy-5,5'-bis(3-methoxycarbonylpropyl)biphenyl, 4.01 ml (1.9851 mmol) of a 0.4956 N methanol solution of sodium methoxide were added and the solvent was evaporated off. The evaporated redidue was dried sufficiently and was dissolved in 5 ml of DMF, to which was added to 0.270 ml (3.309 mmol) of ethyl iodide and the mixture was agitated at room temperature for 4 hours. There to was further added a DMF suspension containing 35.7 mg (0.6617 mmol) of sodium methoxide and the resulting mixture was agitated for 1 hour. The solvent of the reaction mixture was evaporated off under reduced pressure and, to the resulting evaporated residue, an amount of chloroform was added. The solution obtained was washed three times with table salt water and was dried over anhydrous sodium sulfate, before the solvent was evaporated off. The resulting evaporated residue was subjected to a purification by silica gel column chromatography (20 g of WAKO-gel C-200, eluent: toluene and toluene/ethyl acetate of 100/l and 80/l) to obtain 191 mg (0.4321 mol, 65 %) of 2,2'-diethoxy-5,5'-bis(3-methoxycarbonylpropyl) biphenyl as an oily product.

$^1$H-NMR ($CDCl_3$)

δ (ppm) :

1.25 (6H、t、$2CH_3$)、1.95 (4H、m、$2CH_2$)、2.35 (4H、t、$2CH_2$)、2.61 (4H、t、$2CH_2$)、3.66 (6H、s、$2CH_3$)、3.97 (4H、q、$2CH_2$)、6.8-7.2 (6H、m、arom-H)

MS (FAB) : 442 ($M^+$)

Reference Example 6

Synthesis of 2,2'-Dipropoxy-5,5'-bis(3-methoxycarbonylpropyl)biphenyl

To a methanol solution containing 143 mg(0.3350 mmol) of 2,2'-dihydroxy-5,5'-bis(3-methoxycarbonylpropyl)biphenyl, 1.35 ml (0.670 mmol) of a 0.4956 N methanol solution of sodium methoxide were added and the solvent was evaporated off. The evaporated residue was dried sufficiently and was dissolved in 5 ml of DMF, to which was added 0.165 ml (1.675 mmol) of propyl iodide and the mixture was agitated at room temperature for 2 hours. There to was further added a DMF suspension containing 28.2 mg (0.4144 mmol) of sodium methoxide and the resulting mixture was agitated for 1 hour. The solvent of the reaction mixture was evaporated off under reduced pressure and, to the resulting evaporated residue, an amount of chloroform was added. The solution obtained was washed three times with table salt water and was dried over anhydrous sodium sulfate, before the solvent was evaporated off. The resulting evaporated residue was subjected to a purification by silica gel column chromatography (20 g of WAKO-gel C-200, eluent: hexane and hexane/ethyl acetate of 16/l) to obtain 94 mg (0.2000 mmol, 60 %) of 2,2'-dipropoxy -5,5'-bis(3-methoxycarbonylpropyl)biphenyl as an oily product.

$^1$H-NMR ($CDCl_3$)

δ (ppm) :

0.85 (6H、t、$2CH_3$)、1.3-2.1 (8H、m、$2CH_2$、$2CH_2$)、2.1-2.4 (4H、m、$2CH_2$)、2.4-2.7 (4H、m、$2CH_2$)、3.65 (6H、s、$2CH_3$)、3.83 (4H、t、$2CH_2$)、6.7-7.2 (6H、m、arom-H)

MS (FAB) : 470 ($M^+$)

Reference Example 7

Synthesis of 2,2'-Dipentyloxy-5,5'-bis(3-methoxycarbonylpropyl)biphenyl

To 10 ml of a DMF solution containing 400 mg (1.0363 mmol) of 2,2'-dihdroxy-5,5'-bis(3-methoxycarbonylpropyl)biphenyl and 0.530 ml (4.145 mmol) of pentyl bromide, there were added 1.14 ml (7.4613 mmol) of DBU and the resuling mixture was agitated for 2 days at room temperature. Thereto was further added 0.795 ml (6.218 mmol) of pentyl bromides and the mixture was further agitated overnight at 65°C, to which was then added 0.136 ml (1.0363 mmol) of pentyl iodide and the agitation was continued for one night. The solvent of the reaction mixture was evaporated off under reduced pressure, and, to the resulting evaporated residue, and amount of chloroform was added. The obtained solution was washed with dilute hydrochloric acid, water and saline water in this sequence and was dried over anhydrous sodium sulfate The residue obtained after the solvent had been evaporated off was purified by silica gel column chromatography (with 40 g of WAKO-gel C-200, eluent : hexane and hexane/ethyl acetate of 20/l and 10/l) to obtain 313.2 mg (0.5954 mmol, 57 %) of 2,2'-dipentyl-oxy-5,5'-bis(3-methoxycarbonylpropyl)biphenyl as an oily product.

$^1$H-NMR ($CDCl_3$)

δ (ppm) :

0.83 (6H、t、$2CH_3$)、1.0-1.8 (12H、m、$6CH_2$)、1.8-2.1 (4H、m、$2CH_2$)、2.34 (4H、t、$2CH_2$)、2.61 (4H、t、

$2CH_2$)、3.66 (6H、s、$2CH_3$)、3.86 (4H、t、$2CH_2$)、6.7-7.2 (6H、m、arom-H)
MS (FAB) : 526 ($M^+$)

Reference Example 8

Synthesis of 2,2'-Dicyclohexylmethoxy-5,5'-bis-(3-methoxycarbonylpropyl)biphenyl

To 5 ml of a DMF solution containing 250 mg (0.6477 mmol) of 2,2'-dihydroxy-5,5'-bis(3-methoxycarbonylpropyl)biphenyl and 0.920 ml (6.477 mmol) of cyclohexylmethyl bromide, there were added 360 ml (2.5907 mmol) of anhydrous potassium carbonate and a small amount of copper powder and the resulting mixture was agitated overnight at 100°C. The reaction mixture was filtered with suction though Celite to remove the solid matter, which was washed with ethyl acetate. After the solvent in the filtrate had been evaporated off under reduced pressure, the evaporated residue was dissolved in an amount of chloroform. This solution was washed with dilute hydrochloric acid, dilute aqueous sodium bicarbonate, water and saline water in this sequence and was dried over anhydrous sodium sulfate. The residue obtained after the solvent had been evaporated off was purified by silica gel column chromatography (with 12 g of WAKO-gel C-200, eluent :hexane and hexane/ethyl acetate of 25/l) to obtain 224 mg (0.3877 mmol, 60 %) of 2,2'-dicyclohexylmethoxy-5,5'-bis(3-methoxycarbonylpropyl)biphenyl as a white solid.
$^1H$-NMR ($CDCl_3$)
δ (ppm) :
0.6-2.2 (26H、m、cyclohexyl-H、$2CH_2$)、2.34 (4H、t、$2CH_2$)、2.60 (4H、t、$2CH_2$)、3.65 (4H、d、$2CH_2$)、3.66 (6H、s、$2CH_3$)、6.7-7.2 (6H、m、arom-H)
MS (FAB) : 578 ($M^+$)

Example 5

Synthesis of 2,2'-Diethoxy-5,5'-bis-(3-carboxypropyl)biphenyl

To 10 ml of an ethanol solution containing 183.3 mg (0.4147 mmol) of 2,2'-diethoxy- 5,5'-bis(3-methoxycarbonylpropyl)biphenyl obtained in Reference Example 5, there were added 3.3 ml (3.3 mmol) of 1 N sodium hydroxide solution and the mixture was agitated at room temperature for 24 hours. The solvent of the reaction mixture was evaporated off under reduced pressure and, to the resulting evaporated residue, an amount of water was added. The solution obtained was acidified by adding 1 N hydrochloric acid to a pH of 1 - 2. The thus formed precipitate was filtered, washed with water and dried, whereby 164 mg (0.3966 mmol, 96 %) of the title compound were obtained as a white solid.
$^1H$-NMR ($CDCl_3$)
δ (ppm) :
1.25 (6H、t、$2CH_3$)、1.96 (4H、m、$2CH_2$)、2.37 (4H、t、$2CH_2$)、2.64 (4H、t、$2CH_2$)、3.97 (4H、q、$2CH_3$)、6.8-7.2 (6H、m、arom-H)
MS (FAB) : 414 ($M^+$)

Example 6

Synthesis of 2,2'-Dipropoxy-5,5'-bis-(3-carboxypropyl)biphenyl

To 5 ml of a methanol solution containing 93.1 mg (0.2106 mmol) of 2,2'-dipropoxy- 5,5'-bis(3-methoxycarbonylpropyl)biphenyl obtained in Reference Example 6, 1.7 ml (1.7 mmol) of 1N sodium hydroxide solution were added and the mixture was agitated at room temperature for 2 days. The solvent of the reaction mixture was evaporated off under reduced pressure and, to the resulting evaporated residue, an amount of water was added. The solution obtained was washed with ether and was then acidified using 1 N hydrochloric acid and then 0.1 N hydrochloric acid to a pH of about 3. The thus formed precipitate was extracted with ethyl acetate and the extract was washed with water and then with saline water and was then dried over anhydrous sodium sulfate. By evaporating off the solvent, 58.8 mg (0.1330 mmol, 63 %) of the title compound were obtained as a white solid.
$^1H$-NMR ($CDCl_3$)
δ (ppm) :
0.85 (6H、t、$2CH_3$)、1.4-2.2 (8H、m、$2CH_2$, $2CH_2$)、2.37 (4H、t、$2CH_2$)、2.63 (4H、t、$2CH_2$)、3.83 (4H、t、$2CH_2$)、6.7-7.2 (6H、m、arom-H)
MS (FAB) : 441 ($M-H^-$)

Example 7

Synthesis of 2,2'-Dipentyloxy-5,5'-bis-(3-carboxypropyl)biphenyl

To 7 ml of a methanol solution containing 156.6 mg (0.2977 mmol) of 2,2'-dipentyloxy-5,5'-bis(3-methoxycarbonylpropyl)biphenyl obtained in Reference Example 7, 2.4 ml (2.4 mmol) of 1 N sodium hydroxide solution were added and the mixture was agitated at room temperature for 24 hours. The solvent of the reaction mixture was evaporated off under reduced pressure and, to the resulting evaporated residue, an amount of water was added. The solution obtained was washed with ether and was then acidified using 1 N hydrochloric acid and then 0.1 N hydrochloric acid to a pH of about 3. The thus formed precipitate was extracted with ethyl acetate and the extract was washed with water and then with saline water and was then dried over anhydrous sodium sulfate. By evaporating off the solvent, 69.9 mg (0.1404 mmol, 47 %) of the title compound were obtained as a white solid.

$^1$H-NMR ($CDCl_3$)

δ (ppm) :

0.82 (6H、t、$2CH_3$)、1.0-1.8 (12H、m、$6CH_2$)、1.8-2.2 (4H、m、$2CH_2$)、2.36 (4H、t、$2CH_2$)、2.63 (4H、t、$2CH_2$)、3.85 (4H、t、$2CH_2$)、6.7-7.2 (6H、m、arom-H)

MS (FAB) : 498 ($M^+$)

Example 8

Synthesis of 2,2'-Dicyclohexylmethoxy-5,5'-bis-(3-carboxypropyl)biphenyl

To 4 ml of a methanol solution containing 89.9 mg (0.1555 mmol) of 2,2'-dicyclohexylmethoxy-5,5'-bis(3-methoxycarbonylpropyl)biphenyl obtained in Reference Example 8, 1.3 ml (1.3 mmol) of 1 N sodium hydroxide solution were added and the mixture was agitated at room temperature for 4 days. The solvent of the reaction mixture was evaporated off under reduced pressure and, to the resulting evaporated residue, an amount of water was added. The solution obtained was washed with ehter and was then acidified using 1 N hydrochloric acid and then 0.1 N hydrochloric acid to a pH of about 3. The thus formed precipitate was extracted with ethyl acetate and the extract was washed with water and then with saline water and was then dried over anhydrous sodium sulfate. By evaporating off the solvent, 62.7 mg (0.114 mmol, 73 %) of the title compound were obtained as a white solid.

$^1$H-NMR ($CDCl_3$)

δ (ppm) :

0.6-2.2 (26H、m、$2CH_2$、cyclohexyl-H、$2CH_2$)、2.36 (4H、t、$2CH_2$)、2.63 (4H、t、$2CH_2$)、3.65 (4H、d、$2CH_2$)、6.7-7.2 (6H、m、arom-H)

MS (FAB) : 550 (MÛ)

Example 9

Production of Disodium Salt of 2,2'-Dimetoxy-5,5'-bis-(3-carboxypropyl)biphenyl

386 mg (1 mmol) of a known compound 2,2'-dimethoxy-5,5'-bis(3-carboxylpropyl)biphenyl were suspended in 50 ml of distilled water and the compound was dissolved by adding thereto 2 ml of 1 N NaOH aq. solution. The solution Has concentrated by evaporation under reduced pressure and was subjected to freeze-drying, whereby 427 mg of the title compound were obtained.

In the same manner as above, the compounds of formula [I] produced in Examples 2 - 4 and 6 - 8 were able to be converted into disodium salts.

Example 10

Encapsulated Drug

An encapsulated drug product was prepared by encapuslating 200 mg of the mixture of the following composition in each #1 capsule.

| Composition | |
|---|---|
| Disodium salt of 2,2'-dimethoxy-5,5'-bis(3-carboxypropyl)biphenyl | 50 mg |
| Lactose | 50 mg |
| Corn starch | 80 mg |
| Crystallized cellulose | 16 mg |
| Calcium stearate | 4 mg |

Example 11

Production of Disodium Salt of 2,2'-Dimethoxy-5,5'-bis(2-carboxyethyl)biphenyl

358 mg (1 mmol) of the compound 2,2'-dimethoxy-5,5'-bis(2-carboxyethyl)biphenyl obtained in Example 1 were suspended in 50 ml of distilled water and the compound was dissolved by adding thereto 2ml of 1 N NaOH aq. solution. The solution was concentrated by evaporation under reduced pressure and was subjected to freeze-drying, whereby 400 mg of the title compound were obtained.

Example 12

Encapsulated Drug

An encapsulated drug product was prepared in the same manner as in Example 10 except that the disodium salt of 2,2'-dimethoxy-5,5'-bis(2-carboxyethyl) biphenyl was employed instead of using the disodium salt of 2,2'-dimethoxy-5,5'-bis(3-carboxypropyl)biphenyl.

Example 13

Production of Disodium Salt of 2,2'-Diethoxy-5,5'-bis(3-carboxypropyl)biphenyl

414 mg (1 mmol) of the compound 2,2'-diethoxy-5,5'-bis(3-carboxypropyl)biphenyl obtained in Example 5 were suspended in 50 ml of distilled water and the compound was dissolved by adding thereto 2 ml of 1 N NaOH aq. solution. The solution was concentrated by evaporation under reduced pressure and was subjected to freeze-drying, whererby 455 mg of the title compound were obtained.

Example 14

Encapsulated Drug

An encapsulated drug product was prepared in the same manner as in Example 10 except that the disodium salt of 2,2'- diethoxy-5,5'-bis(3-carboxypropyl) biphenyl was employed instead of the disodium salt of 2,2'-dimethoxy- 5,5'-bis(3-carboxypropyl)biphenyl.

## Claims

**Claims for the following Contracting States : AT, BE, CH, DE, DK, FR, GB, IT, LI, LU, NL, SE**

1. An aldose reductase inhibitor composition which comprises a compound of formula (I):

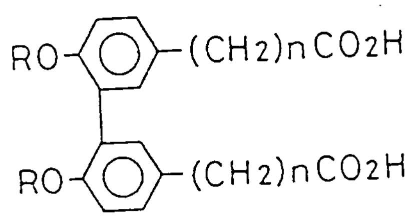

wherein each R, which may be identical or different, is a $C_1$-$C_6$ alkyl group or a cyclohexylmethyl group and each n, which may be identical or different, is 2 or 3, or a physiologically acceptable salt thereof, and a carrier.

2. A composition according to claim 1 wherein the compound of formula (I) is 2,2'-dimethoxy-5,5'-bis(3-carboxypropyl)biphenyl or a physiologically acceptable salt thereof.

3. A composition according to claim 1 wherein the compound of formula (I) is 2,2'-diethoxy-5,5'-bis(3-carboxypropyl)biphenyl or a physiologically acceptable salt thereof.

4. A composition according to claim 1 wherein the compound of formula (I) is 2,2'-diethoxy-5,5'-bis(3-carboxyethyl)biphenyl or a physiologically acceptable salt thereof.

5. A compound of formula (I) as defined in any one of claims 1, 3 or 4 or a physiologically acceptable salt thereof, with the proviso that when the compound is in the form of the free acid, and both R groups are methyl groups, both ns are not 3.

6. A process for the preparation of a compound of formula (I) as defined in any one of claims 1 to 4, which comprises hydrolysing a compound of formula (II):

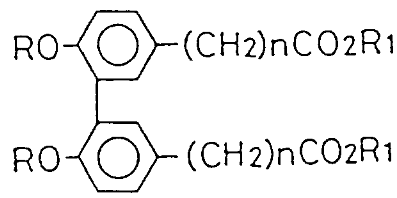

(II)

wherein R and n are as defined in claim 1 and each $R_1$, which may be identical or different, is a radical removable by hydrolysis and, if desired, converting the compound of formula (I) thus produced to a physiologically acceptable salt thereof.

7. A process according to claim 6 wherein the compound of formula (II) has been produced by reacting a compound of formula (III):

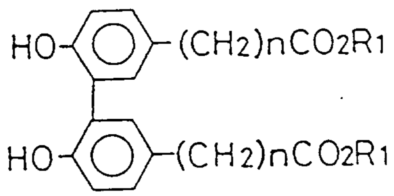

(III)

wherein n and $R_1$ are as defined in claim 6 with a compound of formula (V):

R-X

wherein R is as defined in claim 6 and X is a halogen atom.

8. A compound of formula (I) as defined in any one of claims 1 to 4 or a physiologically acceptable salt thereof for use in a method of treatment of the human or animal body by therapy.

9. A compound of formula (I) as defined in any one of claims 1 to 4 or a physiologically acceptable salt thereof for use in a method of treatment requiring an aldose reductase inhibitor.

10. Use of a compound of formula (I) as defined in any one of claims 1 to 4 or a physiologically acceptable salt thereof in the manufacture of a medicament for the treatment of a condition requiring an aldose reductase inhibitor.

**Claims for the following Contracting States : ES, GR**

1. A process for the preparation of an aldose reductase inhibitor composition which comprises mixing a compound of formula (I):

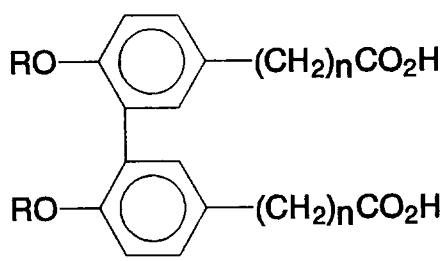

wherein each R, which may be identical or different, is a $C_1$-$C_6$ alkyl group or a cyclohexylmethyl group and each n, which may be identical or different, is 2 or 3, or a physiologically acceptable salt thereof, with a carrier.

2. A process according to claim 1 wherein the compound of formula (I) is 2,2'-dimethoxy-5,5'-bis(3-carboxypropyl)biphenyl or a physiologically acceptable salt thereof.

3. A process according to claim 1 wherein the compound of formula (I) is 2,2'-diethoxy-5,5'-bis(3-carboxypropyl)diphenyl or a physiologically acceptable salt thereof.

4. A process according to claim 1 wherein the compound of formula (I) is 2,2'-diethoxy-5,5'-bis(3-carboxyethyl)biphenyl or a physiologically acceptable salt thereof.

5. A process for the preparation of a compound of formula (I) as defined in any one of claims 1 to 4, which comprises hydrolysing a compound of formula (II):

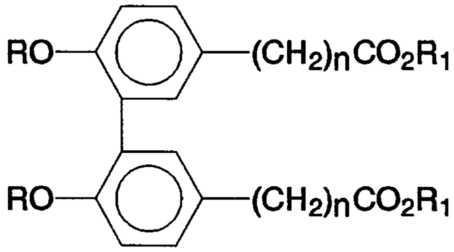

( II )

wherein R and n are as defined in claim 1 and each $R_1$, which may be identical or different, is a radical removable by hydrolysis and, if desired converting the compound of formula (I) thus produced to a physiologically acceptable salt thereof.

6. A process according to claim 5 wherein the compound of formula (II) has been produced by reacting a compound of formula (III):

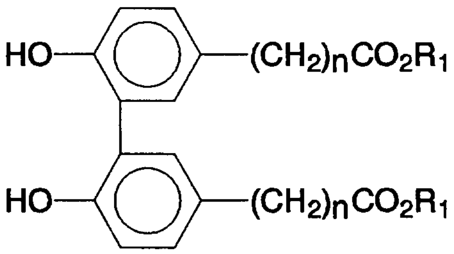

( III )

wherein n and $R_1$ are as defined in claim 5 with a compound of formula (V):

$$R\text{-}X$$

wherein R is as defined in claim 5 and X is a halogen atom.

7. A process as defined in claim 5 or 6 wherein when the compound of formula (I) is in the form of the free acid, and both R groups are methyl groups, both ns are not 3.

8. Use of a compound of formula (I) as defined in any one of claims 1 to 4 or a physiologically acceptable salt thereof in the manufacture of a medicament for the treatment of a condition requiring an aldose reductase inhibitor.

**Patentansprüche**

**Patentansprüche für folgende Vertragsstaaten : AT, BE, CH, DE, DK, FR, GB, IT, LI, LU, NL, SE**

1. Aldosereduktaseinhibitor-Zusammensetzung, umfassend eine Verbindung der Formel (I):

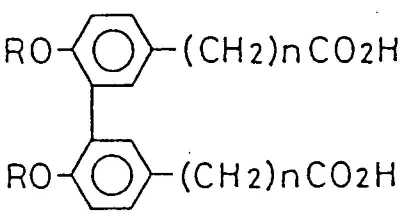

worin jede Gruppe R, die identisch oder verschieden sein kann, eine $C_1$-$C_6$-Alkylgruppe oder eine Cyclohexylmethylgruppe ist und jedes n, das identisch oder verschieden sein kann, 2 oder 3 ist oder eines ihrer physiologisch verträglichen Salze und einen Träger.

2. Zusammensetzung nach Anspruch 1, wobei die Verbindung der Formel (I) 2,2'-Dimethoxy-5,5'-bis-(3-carboxypropyl)-biphenyl oder eines ihrer physiologisch verträglichen Salze ist.

3. Zusammensetzung nach Anspruch 1, wobei die Verbindung der Formel (I) 2,2'-Diethoxy-5,5'-bis-(3-carboxypropyl)-biphenyl oder eines ihrer physiologisch verträglichen Salze ist.

4. Zusammensetzung nach Anspruch 1, wobei die Verbindung der Formel (I) 2,2'-Diethoxy-5,5'-bis-(3-carboxyethyl)-biphenyl oder eines ihrer physiologisch verträglichen Salze ist.

5. Verbindung der Formel (I) gemäß einem der Ansprüche 1, 3 oder 4 oder eines ihrer physiologisch verträglichen Salze, mit der Maßgabe, daß dann, wenn die Verbindung in Form der freien Säure vorliegt und beide Gruppen R Methylgruppen sind, beide n nicht 3 sind.

6. Verfahren zur Herstellung einer Verbindung der Formel (I) gemäß einem der Ansprüche 1 bis 4, bei dem man eine Verbindung der Formel (II) hydrolisiert:

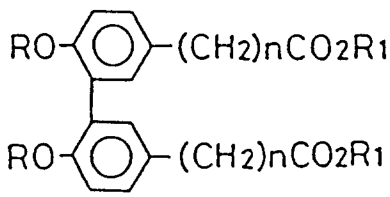

(II)

worin R und n die Bedeutungen gemäß Anspruch 1 besitzen und jede Gruppe $R_1$, die identisch oder verschieden sein kann, ein Radikal ist, das durch Hydrolyse entfernt werden kann, und gegebenenfalls die Verbindung der Formel (I), die auf diese Weise gebildet wurde, in eines ihrer physiologisch verträglichen Salze überführt.

7. Verfahren nach Anspruch 6, wobei die Verbindung der Formel (II) dadurch hergestellt worden ist, daß man eine Verbindung der Formel (III):

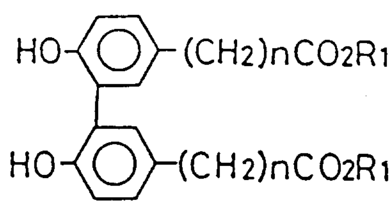

(III)

worin n und $R_1$ die Bedeutungen gemäß Anspruch 6 besitzen, mit einer Verwendung der Formel (V):

R-X

umsetzt, wobei R die Bedeutung gemäß Anspruch 6 besitzt und X ein Halogenatom ist.

8. Verbindung nach Formel (I) gemäß einem der Ansprüche 1 bis 4 oder eines ihrer physiologisch verträglichen Salze zur Verwendung bei einem Verfahren zur therapeutischen Behandlung des menschlichen oder tierischen Körpers.

9. Verbindung der Formel (I) gemäß einem der Ansprüche 1 bis 4 oder eines ihrer physiologisch verträglichen Salze zur Verwendung in einem Verfahren, bei dem eine Behandlung mit einem Aldosereduktaseinhibitor erforderlich ist.

10. Verwendung einer Verbindung der Formel (I) gemäß einem der Ansprüche 1 bis 4 oder eines ihrer physiologisch verträglichen Salze bei der Herstellung eines Medikaments für die Behandlung eines Zustands, der einen Aldosereduktaseinhibitor erfordert.

**Patentansprüche für folgende Vertragsstaaten : ES, GR**

1. Verfahren zur Herstellung einer Aldosereduktaseinhibitor-Zusammensetzung, bei dem man eine Verbindung der folgenden Formel (I):

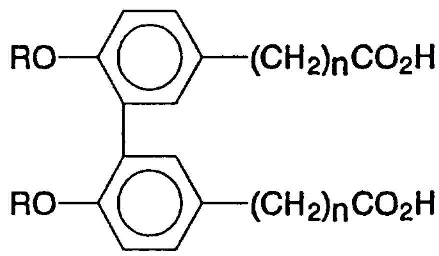

worin jede Gruppe R, die identisch oder verschieden sein kann, eine $C_1$-$C_6$-Alkylgruppe oder eine Cyclohexylmethylgruppe ist und jedes n, das identisch oder verschieden sein kann, 2 oder 3 ist, oder eines ihrer physiologisch verträglichen Salze mit einem Träger mischt.

2. Verfahren nach Anspruch 1, wobei die Verbindung der Formel (I) 2,2'-Dimethoxy-5,5'-bis-(3-carboxypropyl)-biphenyl oder eines ihrer physiologisch verträglichen Salze ist.

3. Verfahren nach Anspruch 1, wobei die Verbindung der Formel (I) 2,2'-Diethoxy-5,5'-bis-(3-carboxypropyl)-biphenyl oder eines ihrer physiologisch verträglichen Salze ist.

4. Verfahren nach Anspruch 1, wobei die Verbindung der Formel (I) 2,2'-Diethoxy-5,5'-bis-(3-carboxyethyl)-biphenyl oder eines ihrer physiologisch verträglichen Salze ist.

5. Verfahren zur Herstellung einer Verbindung der Formel (I) gemäß einem der Ansprüche 1 bis 4, bei dem man eine Verbindung der Formel (II):

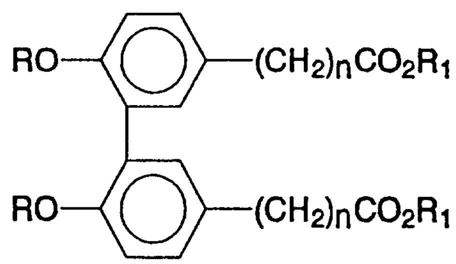

(II)

wobei R und n die Bedeutungen gemäß Anspruch 1 besitzen und jede Gruppe $R_1$, die identisch oder verschieden sein kann, ein Radikal ist, das durch Hydrolyse entfernbar ist, hydrolisiert und gegebenenfalls die Verbindung der Formel (I), die auf diese weise gebildet wurde, in eines ihrer physiologisch verträglichen Salze überführt.

6. Verfahren nach Anspruch 5, bei dem die Verbindung der Formel (II) dadurch hergestellt worden ist, daß man eine Verbindung der Formel (III):

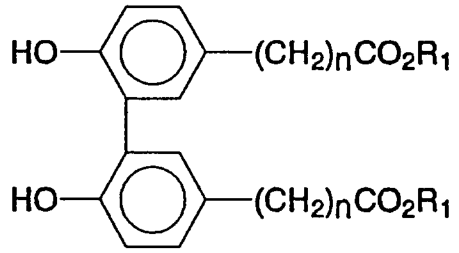

(III)

worin n und $R_1$ die Bedeutungen gemäß Anspruch 5 besitzen, mit einer Verwendung der Formel (V) umsetzt: wobei R die Bedeutung gemäß Anspruch 5 besitzt und X ein Halogenatom ist.

7. Verfahren nach Anspruch 5 oder 6, wobei dann, wenn die Verbindung der Formel (I) in Form der freien Säure vorliegt und beide Gruppen R Methylgruppen sind, beide n nicht 3 sind.

8. Verwendung einer Verbindung der Formel (I) gemäß einem der Ansprüche 1 bis 4 oder eines ihrer physiologisch verträglichen Salze bei der Herstellung eines Medikaments zur Behandlung eines Zustands, der einen Aldosereduktaseinhibitor erfordert.

**Revendications**

**Revendications pour les Etats contractants suivants : AT, BE, CH, DE, DK, FR, GB, IT, LI, LU, NL, SE**

1. Composition inhibitrice de l'aldose réductase qui comprend un composé de formule (I) :

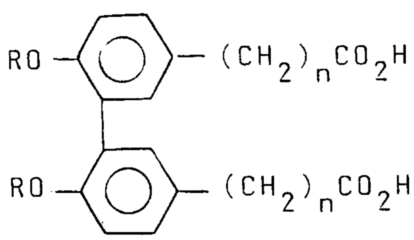

où chaque R, qui peut être identique ou différent, est un groupe alkyle $C_1$-$C_6$ ou un groupe cyclohexylméthyle et chaque n, qui peut être identique ou différent, est 2 ou 3, ou bien un sel physiologiquement acceptable et un support.

2. Composition selon la revendication 1, où le composé de formule (I) est le 2,2'-diméthoxy-5,5'-bis(3-carboxypropyl)biphényle ou son sel physiologiquement acceptable.

3. Composition selon la revendication 1, où le composé de formule (I) est le 2,2'-diéthoxy-5,5'-bis(3-carboxypropyl)biphényle ou son sel physiologiquement acceptable.

4. Composition selon la revendication 1 où le composé de formule (I) est le 2,2'-diéthoxy-5,5'-bis(3-carboxyéthyl)biphényle ou son sel physiologiquement acceptable.

5. Composé de la formule (I), tel que défini selon l'une quelconque des revendications 1, 3 ou 4, ou son sel physiologiquement acceptable, à condition que quand le composé est sous la forme de l'acide libre, et si les deux groupes R sont des groupes méthyles, les deux n ne soient pas 3.

6. Procédé pour la préparation d'un composé de formule (I), tel que défini selon l'une quelconque des revendications 1 à 4, qui consiste à hydrolyser un composé de formule (II) :

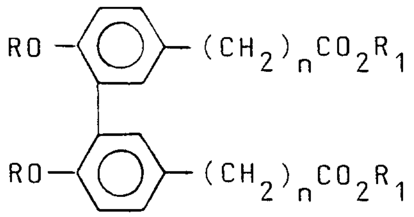

(II)

où R et n sont tels que définis à la revendication 1 et chaque $R_1$, qui peut être identique ou différent, est un radical pouvant être retiré par hydrolyse et, si on le souhaite, à convertir le composé de formule (I) ainsi produit en son sel physiologiquement acceptable.

7. Procédé selon la revendication 6, où le composé de formule (II) a été produit par réaction d'un composé de formule (III) :

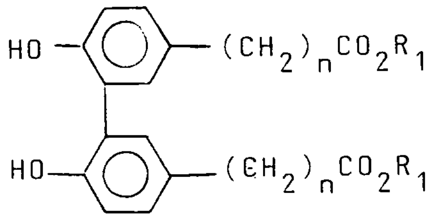

(III)

où n et $R_1$ sont tels que définis à la revendication 6 avec un composé de formule (V) :

R-X

où R est tel que défini à la revendication 6 et X est un atome d'halogène.

8. Composé de formule (I) tel que défini selon l'une quelconque des revendications 1 à 4 ou son sel physiologiquement acceptable pour une utilisation dans une méthode de traitement du corps humain ou animal par thérapie.

9. Composé de la formule (I) tel que défini selon l'une quelconque des revendications 1 à 4 ou son sel physiologiquement acceptable pour une utilisation dans une méthode de traitement nécessitant un inhibiteur de l'aldose réductase.

10. Utilisation d'un composé de formule (I) tel que défini selon l'une quelconque des revendications 1 à 4 ou son sel physiologiquement acceptable dans la fabrication d'un médicament pour le traitement d'une condition nécessitant un inhibiteur de l'aldose réductase.

**Revendications pour les Etats contractants suivants : ES, GR**

1. Procédé pour la préparation d'une composition inhibitrice d'aldose réductase qui consiste à mélanger un composé de formule (I) :

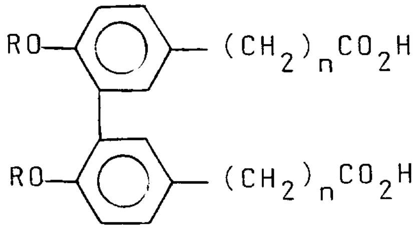

où chaque R, qui peut être identique ou différent, est un groupe alkyle $C_1$-$C_6$ ou un groupe cyclohexylméthyle et chaque n , qui peut être identique ou différent, est 2 ou 3, ou son sel physiologiquement acceptable, avec un support.

2. Procédé selon la revendication 1, où le composé de formule (I) est le 2,2'-diméthoxy-5,5'-bis(3-carboxypropyl)-biphényle ou son sel physiologiquement acceptable.

3. Procédé selon la revendication 1 où le composé de formule (I) est le 2,2'-diéthoxy-5,5'-bis(3-carboxypropyl)-diphényle ou son sel physiologiquement acceptable.

4. Procédé selon la revendication 1 où le composé de formule (I) est le 2,2'-diéthoxy-5,5'-bis(3-carboxyéthyl)-biphényle ou son sel physiologiquement acceptable.

5. Procédé pour la préparation d'un composé de formule (I) tel que défini selon l'une quelconque des revendications 1 à 4, qui consiste à hydrolyser un composé de formule (II) :

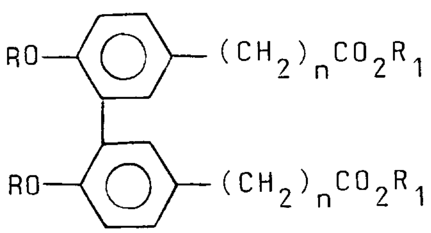

(II)

où R et n sont tels que définis à la revendication 1 et chaque $R_1$, qui peut être identique ou différent, est un radical pouvant être retiré par hydrolyse et, si on le souhaite, à convertir le composé de formule (I) ainsi produit en son sel physiologiquement acceptable.

6. Procédé selon la revendication 5 où le composé de formule (II) a été produit par réaction d'un composé de formule (III) :

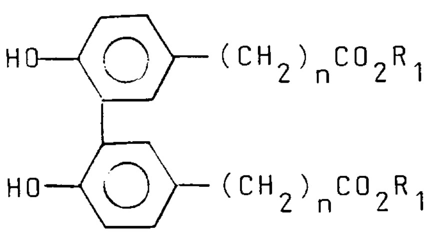

(III)

où n et $R_1$ sont tels que définis à la revendication 5, avec un composé de formule (V) :

R-X

où R est tel que défini à la revendication 5 et X est un atome d'halogène.

7. Procédé tel que défini à la revendication 5 ou 6 où, quand le composé de formule (I) est sous la forme de l'acide libre et si les deux groupes R sont des groupes méthyles,les deux n ne sont pas 3.

8. Utilisation d'un composé de formule (I) tel que défini selon l'une quelconque des revendications 1 à 4, ou d'un sel physiologiquement acceptable de celui-ci, pour la fabrication d'un médicament pour le traitement d'une condition nécessitant un inhibiteur d'aldose réductase.